# EUROPEAN PATENT SPECIFICATION

(11) **EP 3 207 813 B1**
(45) Date of publication and mention of the grant of the patent: **16.12.2020**
(21) Application number: 15850899.4
(22) Date of filing: 14.10.2015
(51) Int. Cl.: A61F 5/02

(54) **BOTTOM WEAR**
UNTERKÖRPERKLEIDUNG
VÊTEMENT POUR PARTIE INFÉRIEURE DU CORPS

(30) Priority: 15.10.2014 JP 2014210502
(43) Date of publication of application: 23.08.2017
(73) Proprietor: Toray Industries, Inc., Tokyo 103-8666 (JP)
(72) Inventor: KASABO, Miki, Otsu-shi Shiga 520-2141 (JP); MIYAMURA, Takako, Tokyo 101-0032 (JP)
(74) Representative: Kador & Partner PartG mbB
(86) International application number: PCT/JP2015/078999
(87) International publication number: WO 2016/060152

(56) References cited:
- JP-A- H1 046 409
- JP-A- H1 046 409
- JP-A- H07 504 590
- JP-A- 2004 238 766
- JP-U- S 604 510
- JP-U- 3 052 914
- JP-U- H0 210 409
- JP-U- S63 167 113
- JP-Y1- S 094 183
- US-A- 5 157 790
- US-A- 5 351 340
- US-A1- 2012 022 418

## Description

### TECHNICAL FIELD

The present invention relates to a bottom wear.

### BACKGROUND ART

Among occupational diseases, low back pain is one of diseases incident to workers during working, and is spreading in various types of occupations. Examples of occupations where there is a high risk of being attacked by low back pain include those relating to transportation business where workers are engaged in handling heavy weight articles and in long-distance driving and medical workers working for assistance of inpatients and treating emergency patients. Recently there is a greatly increasing number of contractions of low back pain in social welfare facilities for nursing of the aged. Further in recent years, personal computers have been spreading into job sites and homes, and a work for operating a mouse and a keyboard for many hours while gazing a display is a new factor of causing low back pain.

The number of persons suffering from low back pain tends to increase as an age becomes older. However, only a few people take a measure at an initial stage of low back pain, and there are not a few people who take no step until the low back pain gets worse. Japan is facing a problem with a decreased working-age population due to low birthrate and longevity, and therefore, there is an increasing number of senior workers of over 65 years old and woman workers in the light of support of a society. Such being the case, importance of maintenance and promotion of health of valuable workers is increasing more than before, and also in case of working uniforms, a viewpoint of keeping health of workers positively becomes important in addition to conventional functions such as safety, easy action and discrimination.

In order to prevent low back pain, it has been considered effective to tighten a waist portion, in particular a portion including an upper part of a pelvis with a waist protection band to increase an abdominal pressure. The purpose of this is to decrease a burden of a waist by forming an abdominal cavity into a shape like a rugby ball to work as a support. However, there is a problem that since such a waist protection band must be worn on a body after taking off clothes, fitting thereof is troublesome and that also when adjusting the fastening of the waist protection band after wearing it, since a degree of the fastening must be adjusted after clothes are taken off or loosened, this work is troublesome. Further it is said that there is a position of a waist portion to be effectively fastened with the waist protection band, and it is recommended that a position including an upper part of a pelvis is fastened with the waist protection band. However, even if the waist protection band is fastened at a proper position when wearing it, there is a problem that the waist protection band is slipped up during various actions of a body. This problem appears remarkably in the case of women having a large difference between the sizes of a waist and a hip. Therefore, for example, in Patent Document 1, there is disclosed a sportswear with a waist-protection tool. In this sportswear with a waist-protection tool, the protection tool for protecting the waist portion is attached to the wear, and therefore, by fastening the circumference of the waist, a burden on the waist can be reduced and the fastening can be adjusted easily, and further since the waist-protection tool is attached to the wear and integrated therewith, slipping up of the waist-protection tool can be inhibited.
Patent Document 2 discloses a firefighter garment having an outer shell with a waist portion covering a lower back area of a wearer and a front closure, an inner liner having a thermal layer and shaped to fit within the outer shell, and a support member attached to the outer shell, for supporting the lumbar region of a wearer. The garment has an integral lumbar support mechanism which can be activated and deactivated as needed. The waist portion 24 of the garment includes a support member 26 which includes upper and lower elastic straps 28, 30, respectively. The straps 28, 30 extend through slits 41, 42 formed in the outer shell which, at the waist portion 24, includes inner retainer squares 43 of shell material stitched to the outer shell. The straps 28, 30 are secured to the rear of the waist portion 24 by stitching 46 and a plurality of oblong, vertically-extending stays 44 are positioned to lie on either side of the lumbar vertebrae of a wearer.

### PRIOR ART DOCUMENT

### Patent Document

Patent Document 1: JP H10-46409 A
Patent Document 2: US 5,157,790 A

### SUMMARY OF THE INVENTION

### PROBLEM TO BE SOLVED BY THE INVENTION

However, in the sportswear with a waist-protection tool described in Patent Document 1, since the waist-protection tool is attached directly to the wear, a position of the waist-protection tool coming into contact with a body is determined unambiguously. Therefore, it is difficult to fasten the tool at an optimum position of a waist by fitting to a width of a waist and a pelvis which vary from person to person. Further in the sportswear with a waist-protection tool, the waist-protection tool is made of a hard metallic material, or a synthetic resin material, and the wear itself is made of a soft fibrous material. Namely, since the sportswear with a waist-protection tool is made of a combination of plural materials having different hardness, a fabric of the wear around the waist-protection tool is deteriorated after repeated laundering. Further, when a tumbler drier is used for drying of the sportswear, the waist-protection tool is deformed, and as a result, a life of the wear itself is decreased. Therefore in order to prevent deterioration of the fabric and deformation of the waist-protection tool which result from the laundering, when the laundering is done by scrubbing with hands, it takes much time and labor for the laundering, which lacks in practicability.

Accordingly, the present invention provides a bottom wear assuring easy adjustment of a waist-fastening position which varies from person to person, practicability saving time and labor in maintenance such as laundering, and easy adjustment of an abdominal pressure enabling a waist portion to be tightened and loosened easily.

### MEANS TO SOLVE THE PROBLEM

In order to achieve the mentioned object, the present invention relates to a bottom wear as set out in claim 1.

Further it is preferable that the bottom wear of the present invention has a means for detachably fixing the waist protection part on the rear inner side of the above-mentioned waist-encircling section.

Further it is preferable that in the bottom wear of the present invention, the above-mentioned fixing means has a means for adjusting a fixing position of the waist protection part.

Further it is preferable that the bottom wear of the present invention has at least one belt loop on the waist-encircling section.

Further it is preferable that in the bottom wear of the present invention, a width of the belt loop in a waist-encircling direction is from 1 cm to 5 cm, and a width of the belt loop in a direction vertical to the waist-encircling direction is from 2.5 cm to 20.5 cm.

Further it is preferable that the bottom wear of the present invention has a third hole and a fourth hole on the waist-encircling section, wherein the third hole and the fourth hole are located nearer to a back side of the waist-encircling section than the first hole and the second hole.

Further it is preferable that in the bottom wear of the present invention, a first surface of the protection band for low back pain faces the inner surface of the waist-encircling section,
the protection band for low back pain has a first belt portion located at the left side of the waist protection part facing the first surface of the protection band for low back pain, a second belt portion located at the right side of the waist protection part facing the first surface of the protection band for low back pain, and a first auxiliary belt and a second auxiliary belt on the first surface of the protection band for low back pain,
the first belt portion has a fifth fixing part,
the second belt portion has a sixth fixing part,
a first end portion of the first auxiliary belt is fixed to the waist protection part, and a second end portion thereof is passed through the third hole and is placed on the outer surface of the waist-encircling section,
a first end portion of the second auxiliary belt is fixed to the waist protection part, and a second end portion thereof is passed through the fourth hole and is placed on the outer surface of the waist-encircling section,
the second end portion of the first auxiliary belt has a seventh fixing part which is detachably fixed to the fifth fixing part or the sixth fixing part, and
the second end portion of the second auxiliary belt has an eighth fixing part which is detachably fixed to the fifth fixing part or the sixth fixing part.

Further it is preferable that the bottom wear of the present invention has a gathered portion on the whole or a part of the waist-encircling section.

Further it is preferable that in the bottom wear of the present invention, a width of the gathered portion in a waist-encircling direction is 2 cm or more.

### EFFECTS OF THE INVENTION

The present invention provides a bottom wear assuring easy adjustment of a waist-fastening position which varies from person to person, practicability saving time and labor in maintenance such as laundering, and easy adjustment of an abdominal pressure enabling a waist portion to be tightened and loosened easily.

### BRIEF DESCRIPTION OF THE DRAWINGS

FIG. 1 is a perspective front view of the neighboring portion of the waist-encircling section in one embodiment of the bottom wear of the present invention.
FIG. 2 is a side view of the neighboring portion of the waist-encircling section of the bottom wear of FIG. 1 when viewing from the first side portion.
FIG. 3 is a front view showing one embodiment of the protection band for low back pain when viewing a surface thereof facing the waist-encircling section.
FIG. 4 is a front view showing the protection band for low back pain shown in FIG. 3 when viewing a surface thereof facing the wearer.
FIG. 5 is a perspective front view of the neighboring portion of the waist-encircling section in one embodiment of the bottom wear where the waist protection part is fixed at a higher position of the waist-encircling section in a direction of a height of the wearer.
FIG. 6 is a perspective front view of the neighboring portion of the waist-encircling section in one embodiment of the bottom wear where the waist protection part is fixed at a lower position of the waist-encircling section in a direction of a height of the wearer.
FIG. 7 is a side view of a peripheral part of the waist-encircling section of the bottom wear shown in FIG. 6 when viewing from the first side portion.
FIG. 8 is a perspective front view of the neighboring portion of the waist-encircling section in another embodiment of the bottom wear of the present invention.
FIG. 9 is a side view of a peripheral part of the waist-encircling section of the bottom wear shown in FIG. 8 when viewing from the first side portion.
FIG. 10 is a front view of one embodiment of the protection band for low back pain having auxiliary belts when viewing a surface of the protection band for low back pain facing the inner surface of the waist-encircling section when the protection band for low back pain is fitted to the bottom wear.
FIG. 11 is a front view of the protection band for low back pain shown in FIG. 10 when viewing the wearer's side surface of the protection band for low back pain fitted to the bottom wear.
FIG. 12 is a side view of a peripheral part of the waist-encircling section of the bottom wear shown in FIG. 8 with the protection band for low back pain shown in FIG. 10 being fitted to the wear when viewing from the first side portion.
FIG. 13 is a side view of the neighboring portion of the waist-encircling section in another embodiment of the bottom wear of the present invention when viewing from the first side portion.
FIG. 14 is a side view of a peripheral part of the waist-encircling section of the bottom wear shown in FIG. 13 with the protection band for low back pain shown in FIG. 10 being fitted to the wear when viewing from the first side portion.
FIG. 15 is a perspective front view of the neighboring portion of the waist-encircling section in another embodiment of the bottom wear of the present invention.

### EMBODIMENT FOR CARRYING OUT THE INVENTION

The bottom wear of the present invention has at least a waist-encircling section, and the waist-encircling section at least has a first hole and a second hole.

Here, the waist-encircling section is a cloth portion of the bottom wear located so-called around the waist ranging from a lower part of a spinal column to a pelvis of a wearer. Namely the waist-encircling section is the whole of the cloth portion of the bottom wear coming into contact with a periphery of a waist portion including a right side, a front and a left side of an abdomen and the waist of the wearer. Further the waist-encircling section includes belt loops, side pockets, back pockets and a waist portion including front and back fasteners. Further in the case of trousers, a rise portion is included in addition to those mentioned above.

Further the bottom wear means whole clothes worn on a lower half of a body including skirts, and trousers such as slacks, jeans, chino pants and cargo pants.

Here, the holes indicate holes penetrating from a surface of a cloth at the side of a wearer's body to an outer surface at the back side thereof, and while a shape of the hole is not limited particularly, as described later, it is desirably cut in a form of vertical slit in order to pull a belt out of the slit smooth when pulling out the belt through the hole from the inner side to the outer side of the waist-encircling section.

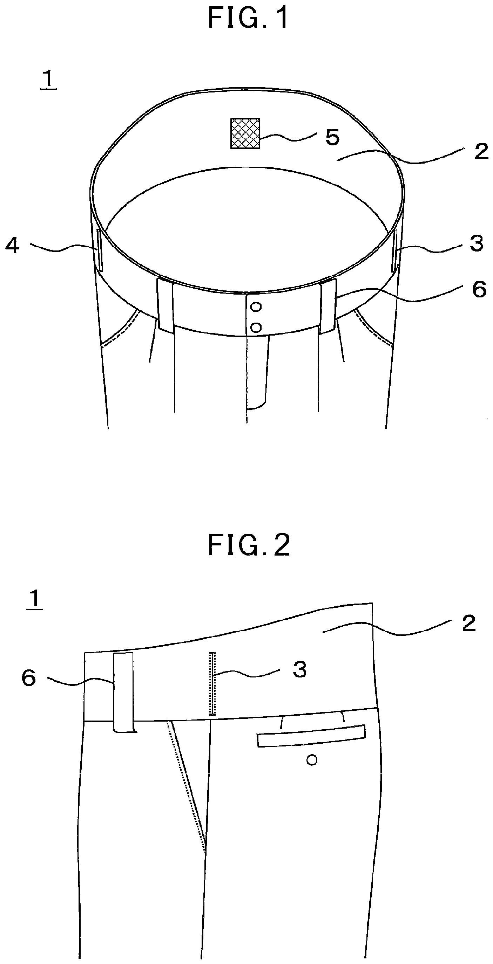

FIG. 1 is a perspective front view of the neighboring portion of the waist-encircling section in one embodiment of the bottom wear of the present invention. The neighboring portion of the waist-encircling section 1 of the bottom wear is provided with the waist-encircling section 2, and this waist-encircling section 2 has the first hole 3 at a first side portion and the second hole 4 at a second side portion.

FIG. 2 is a side view of the neighboring portion of the waist-encircling section of the bottom wear of FIG. 1 when viewing from the first side portion.

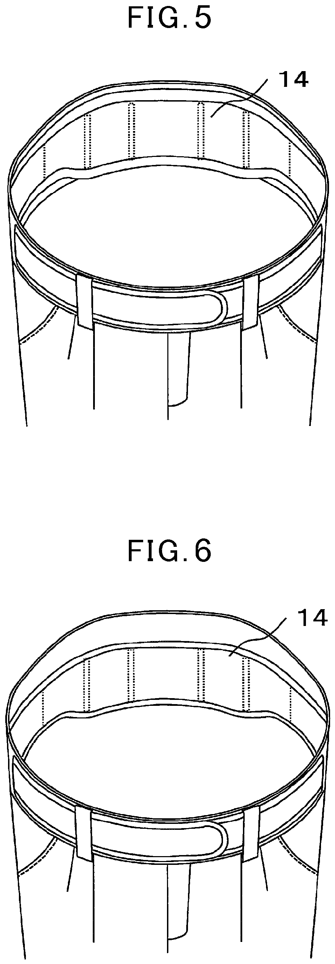

By making such a configuration as mentioned above, a commercially available usual protection band for low back pain can be disposed as shown in FIG. 6 described later and a pressure on a waist portion of a wearer can be adjusted. In that case, while a waist protection part of the protection band for low back pain is disposed on a wearer's side surface of the waist-encircling section, since the waist protection part is not attached to the wear and is not fixed directly, the wearer can easily adjust a waist-fastening position so as to give an optimum pressure on an optimum portion of the wearer.

Further, the two end portions of the protection band for low back pain fitted to the bottom wear of the present invention are passed through the first hole and the second hole and are detachably fixed to each other on an outer surface at the back side of the wearer's side surface of the waist-encircling section (hereinafter referred to as the outer surface). Thus, when adjusting a pressure on a waist portion, the wearer need not take off the bottom wear, which assures easy adjustment of an abdominal pressure.

Further, since the protection band for low back pain is not fixed (e.g. stitched) directly to the bottom wear, in the case of laundering of the bottom wear, the protection band for low back pain can be taken off from the bottom wear. Therefore, even in the case of laundering with washing machine, since the bottom wear and the protection band for low back pain can be laundered separately, a damage on the bottom wear can be inhibited. Accordingly, the bottom wear of the present invention is time- and labor-saving one in maintenance such as laundering and is excellent in practicability. Further, since the protection band for low back pain and the bottom wear are separate parts, the bottom wear which is prone to become stained and has high durability in laundering can be laundered frequently, and the protection band for low back pain which is not high in durability in laundering and is hardly stained as compared with the bottom wear does not need to be laundered frequently. Further, since respective laundering means suitable for the bottom wear and the protection band for low back pain can be used, the bottom wear combined with the protection band for low back pain and having a waist-protecting function is very excellent in durability.

Further, as a wearing period of time becomes longer, wearing out or breakage of the bottom wear easily arises, or loosening of a rubber of the belt portion of the protection band for low back pain is apt to occur. Namely any of these parts may be subject to deterioration. Even in such a case, only a defective part may be replaced with a new one, and therefore the bottom wear of the present invention is economical and is time- and labor-saving in maintenance thereof.

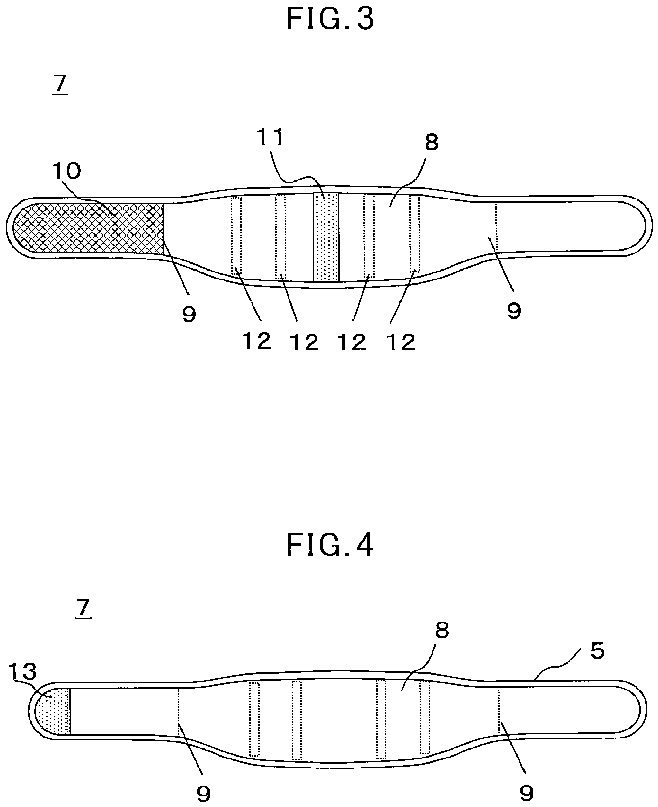

Further, the waist-encircling section 2 of the bottom wear has a first fixing part 5 on its surface at the wearer's side. By making such a configuration, the first fixing part 5 is detachably fixed to a second fixing part of the waist protection part of the protection band for low back pain shown in FIG. 3 explained later, thereby fixing the waist protection part to the waist-encircling section of the bottom wear. Accordingly, it is possible to inhibit the waist protection part from slipping up during various actions of the wearer.

Further, it is preferable that the waist-encircling section 2 has at least one belt loop. The belt loop may be provided on the inner side of the waist-encircling section coming into contact with the wearer's body as a means for adjusting a fixing position of the waist protection part, or may be provided on the outer side of the waist-encircling section, and is provided preferably on the outer side of the waist-encircling section. By passing the belt portion of the protection band for low back pain through the belt loop and fixing it to the wearer's body side, it is possible to prevent, for example, an accident such that the above-mentioned belt portion is caught by a protrusion such as a door knob and the wearer falls down. The belt loop is important for satisfying both of safety and reduction of low back pain, in particular when the bottom wear is used as a working uniform.

Here, a width of the belt loop in a waist-encircling direction is preferably from 1 cm to 5 cm, and a width of the belt loop in a direction vertical to the waist-encircling direction is preferably from 2.5 cm to 20.5 cm. When the width of the belt loop in the waist-encircling direction is 1 cm or more, the belt loop is inhibited from being taken off from the bottom wear due to a shock applied to the belt loop. When the width of the belt loop in the waist-encircling direction is 5 cm or less, the flexibility of the waist-encircling section can be made more satisfactory and the bottom wear becomes more excellent in workability. Further, when the width of the belt loop in a direction vertical to the waist-encircling direction is 2.5 cm or more, it becomes easier to pass the belt portion through the belt loop. When the width of the belt loop in a direction vertical to the waist-encircling direction is 20.5 cm or less, the flexibility of the waist-encircling section can be made more satisfactory and the bottom wear becomes more excellent in workability.

Next, the protection band for low back pain will be explained. FIG. 3 is a front view showing one embodiment of the protection band for low back pain when viewing a surface thereof facing the waist-encircling section. The protection band for low back pain 7 has a waist protection part 8, belt portions 9 positioned at right and left sides of the waist protection part 8, a third fixing part 10 positioned at one end portion of the protection band for low back pain, a second fixing part 11 positioned on the waist protection part 8 and bones 12 positioned on the waist protection part 8. Further, FIG. 4 is a front view showing the protection band for low back pain shown in FIG. 3 when viewing a surface thereof facing the wearer. The protection band for low back pain 7 has a waist protection part 8, belt portions 9 positioned at right and left sides of the waist protection part 8, a fourth fixing part 13 positioned on an end portion oppositely to the end portion where the third fixing part 10 is positioned, and bones positioned on the waist protection part 8.

Here, the waist protection part is a wide band which may be made of a material being the same as or different from that of the belt portion. Further a back width of the waist protection part which is a width in a direction of a height of the wearer is preferably from 5 cm to 20 cm. When the back width is 5 cm or more, a fastening pressure on a waist portion of the wearer is concentrated on a narrow width portion and becomes relatively high, thereby enabling a blood vessel to be inhibited from being excessively compressed, and further, in positioning of the waist protection part, a burden for positioning the waist protection part on an upper part of a pelvis can be reduced. When the back width is 20 cm or less, oppressive heat can be reduced when wearing the bottom wear for many hours. In consideration of these points mentioned above, in order to allow the bottom wear to be used for many hours and secure workability while minimizing a limitation in a range of motion, it is preferable to set the back width of the waist protection part to be within the mentioned range. Further, from the viewpoint mentioned above, it is more preferable that a lower limit of the back width is 8 cm or more, and an upper limit of the back width is 15 cm or less.

Further, it is preferable that the waist protection part is provided with bones for stabilizing a position of a spinal column. Here, a material of the bones may be any of a metal, or a resin, and a shape thereof may be any of a spiral, and a strip.

Also, it is further preferable that the waist protection part is provided with a non-slip tape on its wearer's side surface to keep its suitable position. Here, example of the non-slip tape is, for instance, a tape having a filamentous elastic body which has a slip-preventing effect and is located so as to be oriented and protruded in a longitudinal direction of the tape.

Further, it is preferable that the bottom wear of the present invention has a means for detachably fixing the waist protection part at the rear inner side of the waist-encircling section. Here, examples of the means for detachably fixing the waist protection part include the first fixing part provided on the rear inner side of the waist-encircling section of the bottom wear. The first fixing part is fixed detachably to the second fixing part of the waist protection part to fix the waist protection part to the waist-encircling section of the bottom wear, thereby inhibiting the waist protection part from being slipped up due to various actions of the wearer. Here, examples of the first fixing part and the second fixing part include a pair of hook-and-loop fasteners, a pair of buttons such as a pair of snap buttons, a pair of hooks or loops for detachably fixing the waist protection part to the waist-encircling section of the bottom wear. These means can be used alone or a combination of the plural means selected from those mentioned above can also be used.

Further, it is preferable that the above-mentioned fixing means has a means for adjusting the fixing position of the waist protection part. Here, FIG. 5 is a perspective view of the neighboring portion of the waist-encircling section in one embodiment of the bottom wear where the waist protection part is fixed at a higher position of the waist-encircling section in a direction of a height of the wearer. In this bottom wear, the waist protection part 14 is fixed at a higher position of the waist-encircling section in a direction of a height of the wearer. Further, FIG. 6 is a perspective view of the neighboring portion of the waist-encircling section of one embodiment of the bottom wear where the waist protection part is fixed at a lower position of the waist-encircling section in a direction of a height of the wearer. In this bottom wear, the waist protection part 14 is fixed at a lower position of the waist-encircling section in a direction of a height of the wearer. Namely, in these bottom wears, the waist protection part of the protection band for low back pain can be adjusted so as to be fixed at a desired position in the vertical direction of a height of the wearer.

Further, when loops, buttons or hooks (hereinafter referred to as loops) are used as the above-mentioned fixing means, the following means for adjusting the fixing position of the waist protection part can be exemplified. For example, there is an embodiment such that one button is provided on the first fixing part, a plurality of button holes are provided on the second fixing part, and the plurality of button holes are aligned in a direction of a height of a wearer. In this embodiment, in order to fix the waist protection part at a desired position, the wearer selects a button hole for passing one button from the plurality of button holes. The adjusting means is not limited to this embodiment. Also an embodiment of plural buttons and one button hole may be employed, and an embodiment of plural buttons and plural button holes may be employed. Further, there may be employed an embodiment of providing a button hole on the first fixing part and providing a button on the second fixing part.

Further, when a hook-and-loop fastener is used as the above-mentioned fixing means, the following means for adjusting the fixing position of the waist protection part can be exemplified. For example, there is an embodiment such that one of a pair of hook-and-loop fasteners is provided on the first fixing part, the other one of the pair of hook-and-loop fasteners is provided on the second fixing part, a width of the hook-and-loop fastener of the first fixing part side is short in a direction of a height of the wearer, and a width of the hook-and-loop fastener of the second fixing part side is long in a direction of a height of the wearer. In this embodiment, in order to fix the waist protection part at a desired position, the wearer fixes the hook-and-loop fastener of the first fixing part side at an optional position of the hook-and-loop fastener of the second fixing part side in a direction of a height of the wearer. This adjusting means is not limited to such an embodiment. The embodiment of the adjusting means may be such that a width of the hook-and-loop fastener of the first fixing part side is long in a direction of a height of the wearer, and a width of the hook-and-loop fastener of the second fixing part side is short in a direction of a height of the wearer, or that widths of both of the hook-and-loop fasteners of the first fixing part side and the second fixing part side are long in a direction of a height of the wearer.

Among the means for adjusting the position where the waist protection part is located and fixed, use of the hook-and-loop fastener is preferable from the viewpoint of enabling fine adjustment and fixing of the position for placing the waist protection part.

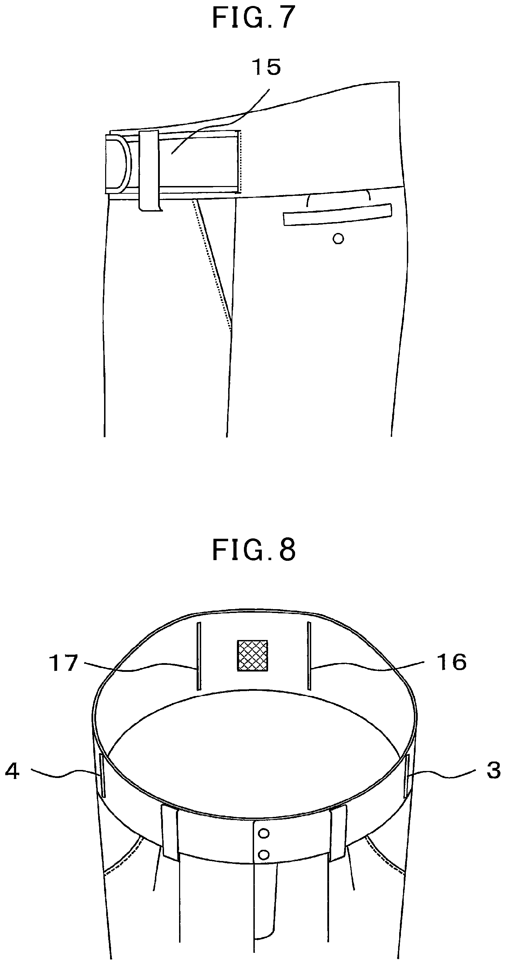

Further, FIG. 7 is a side view of a peripheral part of the waist-encircling section of the bottom wear shown in FIG. 6 when viewing from the first side portion. At the first side portion in the peripheral part of the waist-encircling section of the bottom wear, the belt portion 15 of the protection band for low back pain is passed through the first hole and a part thereof comes out on the outer surface of the bottom wear, and one end portion of the protection band for low back pain is fixed to the other end portion of the protection band for low back pain at the front part of the waist-encircling section of the bottom wear.

FIG. 8 is a perspective front view of the neighboring portion of the waist-encircling section in another embodiment of the bottom wear of the present invention. This bottom wear is suitable for fitting a protection band for low back pain having two auxiliary belts which is to be explained later, and in addition to the first hole 3 and the second hole 4, has a third hole 16 and a fourth hole 17 at the back side of the waist-encircling section when viewing from the first hole 3 and the second hole 4, respectively.

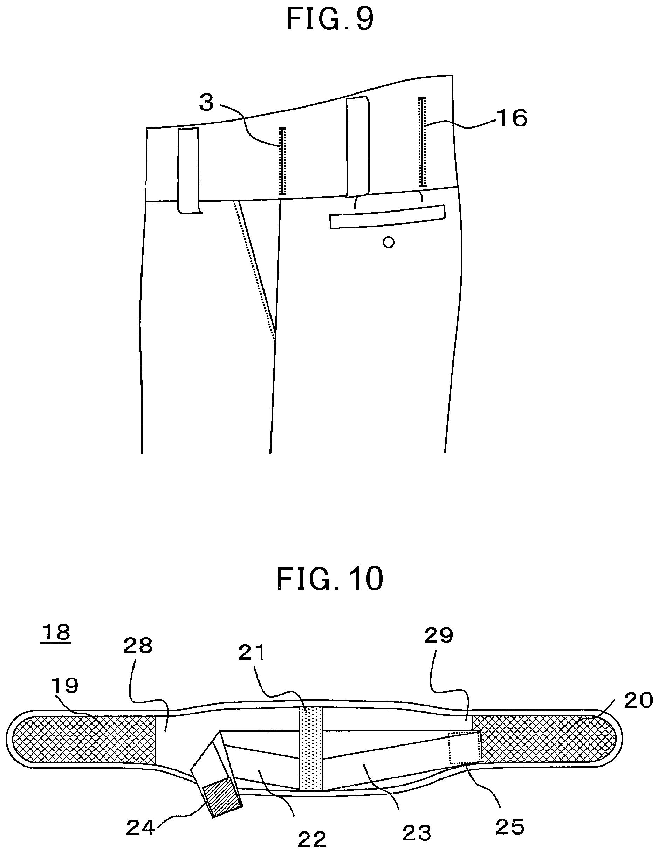

FIG. 9 is a side view of a peripheral part of the waist-encircling section of the bottom wear shown in FIG. 8 when viewing from the first side portion. This bottom wear has the third hole 16 at further back side of the waist-encircling section when viewing from the first hole 3.

It is preferable that the protection band for low back pain fitted to the bottom wear of the present invention has at least one auxiliary belt. One end portion of the auxiliary belt is fixed to the waist protection part, and the other end portion thereof has a fixing part being capable of fixing detachably to the back surface of the protection band for low back pain oppositely to the surface thereof at the wearer's side. By making such a configuration, the number of means for adjusting a pressure on the waist portion of the wearer is increased, and more delicate adjustment of a pressure on the waist portion of the wearer can be made.

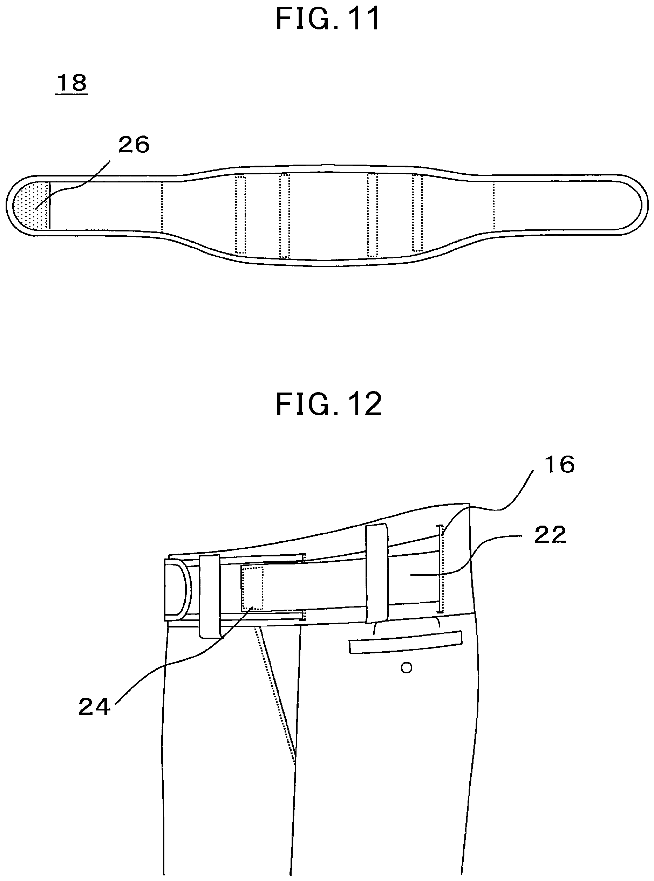

FIG. 10 is a front view of one embodiment of the protection band for low back pain having auxiliary belts when viewing a surface of the protection band for low back pain facing the inner surface of the waist-encircling section when the protection band for low back pain is fitted to the bottom wear. Here, the inner surface of the waist-encircling section means a surface of the waist-encircling section located at the wearer's side when the bottom wear is worn. The protection band for low back pain 18 has the waist protection part, belt portions located at the right and left sides of the waist protection part (the first belt portion 28 and the second belt portion 29), a fifth fixing part 19 located on the first belt portion of the protection band for low back pain, a sixth fixing part 20 located on the second belt portion of the protection band for low back pain, a second fixing part 21 located on the waist protection part and two auxiliary belts (a first auxiliary belt 22 and a second auxiliary belt 23). The first end portion of the first auxiliary belt 22 is fixed to the center portion of the waist protection part, and the second end portion of the first auxiliary belt 22 has a seventh fixing part 24 on its surface at the side of the protection band for low back pain. Also, the first end portion of the second auxiliary belt 23 is fixed to the center portion of the waist protection part, and the second end portion of the second auxiliary belt 23 has an eighth fixing part 25 on its surface at the side of the protection band for low back pain. Further, FIG. 11 is a front view of the protection band for low back pain shown in FIG. 10 when viewing the wearer's side surface of the protection band for low back pain fitted to the bottom wear. The protection band for low back pain 18 has a waist protection part, belt portions located at the right and left sides of the waist protection part (a first belt portion and a second belt portion), a ninth fixing part 26 located on the second belt portion and bones disposed on the waist protection part. In addition, in the protection band for low back pain in this embodiment, the sixth fixing part is provided on one surface of the second belt portion, and on the other surface of the second belt portion is provided the ninth fixing part.

Here, the fifth fixing part 19 can be fitted detachably to the ninth fixing part 26. Further the fifth fixing part 19 can be fixed detachably to the seventh fixing part 24. Furthermore, the sixth fixing part 20 can be fixed detachably to the eighth fixing part 25.

FIG. 12 is a side view of a peripheral part of the waist-encircling section of the bottom wear shown in FIG. 8 with the protection band for low back pain shown in FIG. 10 being fitted to the wear when viewing from the first side portion. In this bottom wear, a part of the first auxiliary belt is passed through the third hole 16 and comes out on the outer surface of the bottom wear. The end portion of the first auxiliary belt coming out on the outer surface of the bottom wear is detachably fixed to the fifth fixing part 19 of the first belt portion of the protection band for low back pain by means of the seventh fixing part 24 provided on the first auxiliary belt.

FIG. 10 illustrates an embodiment of the first end portion of the first auxiliary belt and the first end portion of the second auxiliary belt being fixed at the center portion of the waist protection part. The fixing positions of the first end portion of the first auxiliary belt and the first end portion of the second auxiliary belt are not limited particularly as far as a pressure on the waist part of the wearer can be adjusted. From the viewpoint of a satisfactory adjustable range of the pressure on the waist part of the wearer, the first end portion of the first auxiliary belt and the first end portion of the second auxiliary belt are fixed preferably to the waist protection part, more preferably to the center portion of the waist protection part as shown in FIG. 10.

Further, in FIG. 10, the first auxiliary belt and the second auxiliary belt are arranged at the right and left sides of the center portion of the waist protection part, but the arrangement of the auxiliary belts also is not particularly limited thereto as far as a pressure on the waist part of the wearer can be adjusted. For example, there may be used an embodiment such that the respective first end portions of the first auxiliary belt having a length of one fourth of a length of a perimeter of the waist-encircling section and the second auxiliary belt having a length of seven fourth of the length of the perimeter of the waist-encircling section are fixed at the same position on the waist protection part, and both of the first auxiliary belt and the second auxiliary belt are arranged at the left side of the center portion of the waist protection part. In this case, the second end portion of the first auxiliary belt is fixed to the fifth fixing part, and the second end portion of the second auxiliary belt is fixed to the sixth fixing part. Particularly from the viewpoint of simple configuration and easy adjustment of a pressure on a waist, it is preferable that the first auxiliary belt and the second auxiliary belt are arranged at the right and left sides, respectively of the center portion of the waist protection part.

In the bottom wear shown in FIG. 12, the second end portion of the first auxiliary belt is fixed to the fifth fixing part of the first belt portion of the protection band for low back pain. The bottom wear is not limited to such an embodiment, and there may be employed an embodiment such that by using an auxiliary belt made of a material having a high extensibility, the second end portion of the first auxiliary belt is stretched more in a waist-encircling direction to extend the auxiliary belt, thereby allowing the end portion of the first auxiliary belt to be fixed to the sixth fixing part of the second belt portion of the protection band for low back pain.

Here, the bottom wear shown in FIG. 8 is suitable for fitting, thereto, the protection band for low back pain having the auxiliary belts as shown in FIG. 10. As compared with the case of the bottom wear shown in FIG. 2 wherein a part of the first auxiliary belt is taken out on the outer surface of the waist-encircling section from the first hole of the bottom wear, when a part of the first auxiliary belt is taken out on the outer surface of the waist-encircling section from the third hole as shown in FIG. 12, the auxiliary belt portion which can be caught by the wearer is increased and a pressure on a waist portion can be adjusted easily when the pressure is adjusted with the auxiliary belt.

Further, though not shown in the drawings, in the second auxiliary belt, too, as compared with the case of the bottom wear shown in FIG. 2 wherein a part of the second auxiliary belt is taken out on the outer surface of the waist-encircling section from the second hole of the bottom wear, when a part of the second auxiliary belt is taken out on the outer surface of the waist-encircling section from the fourth hole, the auxiliary belt portion which can be caught by the wearer is increased and a pressure on a waist portion can be adjusted more easily when the pressure is adjusted with the auxiliary belt.

Also, since the protection band for low back pain is provided with the auxiliary belts, the number of means for adjusting the pressure on the wearer's waist is increased from one to three, and therefore, more delicate adjustment of a pressure on the waist portion of the wearer can be made.

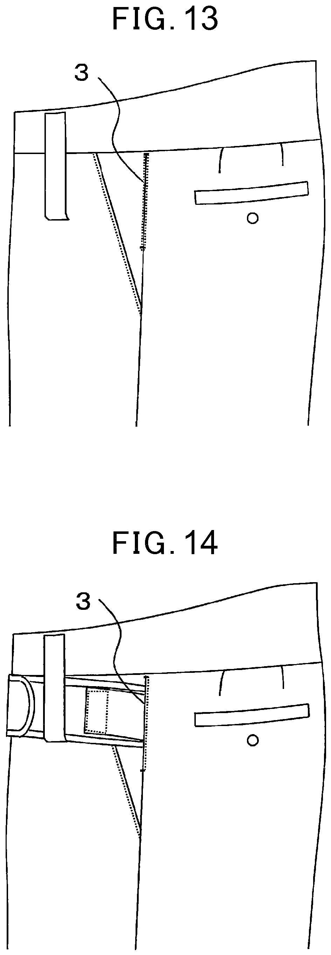

FIG. 13 is a side view of the neighboring portion of the waist-encircling section in another embodiment of the bottom wear of the present invention when viewing from the first side portion. In this bottom wear, as compared with the bottom wear shown in FIG. 1, the first hole 3 provided on the waist-encircling part is disposed at a lower position in a direction of a height of the wearer.

FIG. 14 is a side view of a peripheral part of the waist-encircling section of the bottom wear shown in FIG. 13 with the protection band for low back pain shown in FIG. 10 being fitted to the wear when viewing from the first side portion. A part of the belt portion of the protection band for low back pain and a part of the auxiliary belt are taken out on the outer surface of the waist-encircling section from the first hole 3 provided on the waist-encircling section.

The bottom wear of the present invention may have a gathered portion on the whole or a part of the waist-encircling section. When the waist-encircling section has the gathered portion, cloth bulges and looks three-dimensional, and therefore, fitness to the waist portion in line with a body of the wearer can be enhanced. Here, it is preferable that a width of the gathered portion in a waist-encircling direction is 2 cm or more, from a point of obtaining a stereoscopic effect with the gathered portion. Further it is preferable that the whole part of the waist-encircling section has the gathered portion, and there is no particular restriction in an upper limit of a width of the gathered portion in a waist-encircling direction.

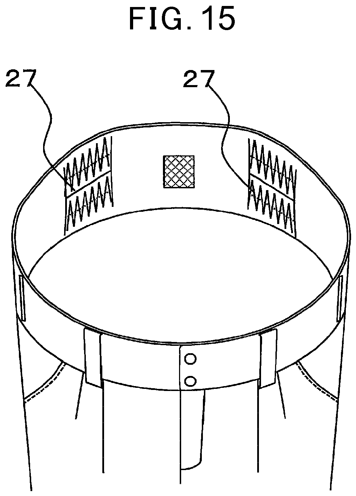

FIG. 15 is a perspective front view of the neighboring portion of the waist-encircling section in another embodiment of the bottom wear of the present invention. This bottom wear has gathered portions 27 on the rear side of its waist-encircling section.

### EXPLANATION OF SYMBOLS

- 1:: Neighboring portion of waist-encircling section
- 2:: Waist-encircling section
- 3:: First hole
- 4:: Second hole
- 5:: First fixing portion
- 6:: Belt loop
- 7:: Protection band for low back pain
- 8:: Waist protection part
- 9:: Belt portion
- 10:: Third fixing portion
- 11:: Second fixing portion
- 12:: Bone
- 13:: Fourth fixing portion
- 14:: Waist protection part
- 15:: Belt portion
- 16:: Third hole
- 17:: Fourth hole
- 18:: Protection band for low back pain
- 19:: Fifth fixing portion
- 20:: Sixth fixing portion
- 21:: Second fixing portion
- 22:: First auxiliary belt
- 23:: Second auxiliary belt
- 24:: Seventh fixing portion
- 25:: Eighth fixing portion
- 26:: Ninth fixing portion
- 27:: Gathered portion
- 28:: First belt portion
- 29:: Second belt portion

## Claims

1. A bottom wear having at least a waist-encircling section (2), wherein the waist-encircling section (2) at least has a first hole (3) and a second hole (4), and the bottom wear further having a protection band for low back pain (7) provided with a waist-protection part (8),
wherein the waist protection part (8) is disposed on a rear inner side of the waist-encircling section (2),
one end portion of the protection band for low back pain (7) is passed through the first hole (3) and placed on an outer surface of the waist-encircling section (2),
the other end portion of the protection band for low back pain (7) is passed through the second hole (4) and placed on an outer surface of the waist-encircling section (2), and
the one end portion of the protection band for low back pain (7) is detachably fixed to the other end portion of the protection band for low back pain (7),
**characterized in that**
the protection band for low back pain (7) is not fixed directly to the bottom wear.

2. The bottom wear of claim 1 having a means for detachably fixing the waist protection part (8) on the rear inner side of the waist-encircling section (2).

3. The bottom wear of claim 2, wherein the fixing means has a means for adjusting a fixing position of the waist protection part (8).

4. The bottom wear of any one of claims 1 to3, having at least one belt loop (6) on the waist-encircling section (2).

5. The bottom wear of claim 4, wherein a width of the belt loop (6) in a waist-encircling direction is from 1 cm to 5 cm, and a width of the belt loop (6) in a direction vertical to the waist-encircling direction is from 2.5 cm to 20.5 cm.

6. The bottom wear of any one of claims 1 to 5, having a third hole (16) and a fourth hole (17) on the waist-encircling section (2), wherein the third hole (16) and the fourth hole (17) are located nearer to a rear side of the waist-encircling section (2) than the first hole (3) and the second hole (4).

7. The bottom wear of claim 6, wherein a first surface of the protection band for low back pain (7) faces the inner surface of the waist-encircling section (2),
the protection band for low back pain has a first belt portion (28) located at the left side of the waist protection part (8,14) facing the first surface of the protection band for low back pain (7), a second belt portion (29) located at the right side of the waist protection part (8,14) facing the first surface of the protection band for low back pain (7), and a first auxiliary belt (22) and a second auxiliary belt (23) on the first surface of the protection band for low back pain (7),
the first belt portion (28) has a fifth fixing part (19),
the second belt portion (29) has a sixth fixing part (20),
a first end portion of the first auxiliary belt (22) is fixed to the waist protection part (8,14), and a second end portion thereof is passed through the third hole (16) and is placed on the outer surface of the waist-encircling section (2),
a first end portion of the second auxiliary belt (23) is fixed to the waist protection part (8,14), and a second end portion thereof is passed through the fourth hole (17) and is placed on the outer surface of the waist-encircling section (2),
the second end portion of the first auxiliary belt (22) has a seventh fixing part (24) which is detachably fixed to the fifth fixing part (19) or the sixth fixing part (20), and
the second end portion of the second auxiliary belt (23) has an eighth fixing part (25) which is detachably fixed to the fifth fixing part (19) or the sixth fixing part (20).

8. The bottom wear of any one of claims 1 to 7, having a gathered portion (27) on the whole or a part of the waist-encircling section (2).

9. The bottom wear of claim 8, wherein a width of the gathered portion (27) in a waist-encircling direction is 2 cm or more.

## Patentansprüche

1. Unterbekleidung, die mindestens einen die Taille umgebenden Abschnitt (2) aufweist, wobei der die Taille umgebende Abschnitt (2) mindestens ein erstes Loch (3) und ein zweites Loch (4) aufweist, und die Unterbekleidung ferner ein Schutzband für Schmerzen im unteren Rücken (7) aufweist, das mit einem Taillenschutzteil (8) ausgestattet ist,
wobei der Taillenschutzteil (8) an einer hinteren Innenseite des die Taille umgebenden Abschnitts (2) angeordnet ist,
wobei ein Endabschnitt des Schutzbandes für Schmerzen im unteren Rücken (7) durch das erste Loch (3) geführt und auf einer Außenoberfläche des die Taille umgebenden Abschnitts (2) angeordnet ist,
wobei der andere Endabschnitt des Schutzbandes für Schmerzen im unteren Rücken (7) durch das zweite Loch (4) geführt und an einer äußeren Oberfläche des die Taille umgebenden Abschnitts (2) angeordnet ist, und
wobei der eine Endabschnitt des Schutzbandes für Schmerzen im unteren Rücken (7) lösbar an dem anderen Endabschnitt des Schutzbandes für Schmerzen im unteren Rücken (7) befestigt ist,
**dadurch gekennzeichnet, dass**
das Schutzband für Schmerzen im unteren Rücken (7) nicht direkt an der Unterbekleidung befestigt ist.

2. Unterbekleidung nach Anspruch 1, umfassend Mittel zum lösbaren Befestigen des Taillenschutzteils (8) an der hinteren Innenseite des die Taille umgebenden Abschnitts (2).

3. Unterbekleidung nach Anspruch 2, wobei das Befestigungsmittel ein Mittel zum Einstellen einer Befestigungsposition von dem Taillenschutzteil (8) aufweist.

4. Unterbekleidung nach einem der Ansprüche 1 bis 3, umfassend mindestens eine Gürtelschlaufe (6) an dem die Taille umgebenden Abschnitt (2).

5. Unterbekleidung nach Anspruch 4, wobei die Breite der Gürtelschlaufe (6) in einer die Taille umgebenden Richtung 1 cm bis 5 cm und die Breite der Gürtelschlaufe (6) in einer Richtung senkrecht zu der die Taille umgebenden Richtung 2,5 cm bis 20,5 cm beträgt.

6. Unterbekleidung nach einem der Ansprüche 1 bis 5, umfassend ein drittes Loch (16) und ein viertes Loch (17) an dem die Taille umgebenden Abschnitt (2), wobei das dritte Loch (16) und das vierte Loch (17) näher an einer Rückseite des die Taille umgebenden Abschnitts (2) angeordnet sind als das erste Loch (3) und das zweite Loch (4).

7. Unterbekleidung nach Anspruch 6, wobei eine erste Oberfläche des Schutzbandes für Schmerzen im unteren Rücken (7) der Innenoberfläche des die Taille umgebenden Abschnitts (2) zugewandt ist,
wobei das Schutzband für Schmerzen im unteren Rücken einen ersten Gürtelabschnitt (28), der an der linken Seite des Taillenschutzteils (8, 14) angeordnet und der ersten Oberfläche des Schutzbandes für Schmerzen im unteren Rücken (7) zugewandt ist, einen zweiten Gürtelabschnitt (29), der an der rechten Seite des Taillenschutzteils (8, 14) angeordnet und der ersten Oberfläche des Schutzbandes für Schmerzen im unteren Rückenbereich (7) zugewandt ist, und einen ersten Hilfsgürtel (22) und einen zweiten Hilfsgürtel (23) auf der ersten Oberfläche des Schutzbandes für Schmerzen im unteren Rückenbereich (7) aufweist,
wobei der erste Gürtelabschnitt (28) einen fünften Befestigungsteil (19) aufweist,
wobei der zweite Gürtelabschnitt (29) einen sechsten Befestigungsteil (20) aufweist,
wobei ein erster Endabschnitt des ersten Hilfsgürtels (22) an dem Taillenschutzteil (8, 14) befestigt ist und ein zweiter Endabschnitt davon durch das dritte Loch (16) geführt und an der Außenoberfläche des die Taille umgebenden Abschnitts (2) angeordnet ist,
wobei ein erster Endabschnitt des zweiten Hilfsgürtels (23) an dem Taillenschutzteil (8, 14) befestigt ist und ein zweiter Endabschnitt davon durch das vierte Loch (17) geführt und auf der Außenoberfläche des die Taille umgebenden Abschnitts (2) angeordnet ist,
wobei der zweite Endabschnitt des ersten Hilfsgürtels (22) einen siebten Befestigungsteil (24) aufweist, das lösbar an dem fünften Befestigungsteil (19) oder dem sechsten Befestigungsteil (20) befestigt ist, und
wobei der zweite Endabschnitt des zweiten Hilfsgürtels (23) einen achten Befestigungsteil (25) aufweist, das lösbar an dem fünften Befestigungsteil (19) oder dem sechsten Befestigungsteil (20) befestigt ist.

8. Unterbekleidung nach einem der Ansprüche 1 bis 7, umfassend einen gerafften Abschnitt (27) an dem gesamten oder einem Teil des die Taille umgebenden Abschnitts (2).

9. Unterbekleidung nach Anspruch 8, wobei die Breite des gerafften Abschnitts (27) in einer die Taille umgebenden Richtung 2 cm oder mehr beträgt.

## Revendications

1. Vêtement pour partie inférieure du corps ayant au moins une section encerclant la taille (2), dans lequel la section encerclant la taille (2) a au moins un premier trou (3) et un deuxième trou (4), et le vêtement pour partie inférieure du corps ayant une bande de protection pour les douleurs lombaires (7) prévue avec une partie de protection de taille (8),
dans lequel la partie de protection de taille (8) est disposée sur un côté interne arrière de la section encerclant la taille (2),
une partie d'extrémité de la bande de protection pour les douleurs lombaires (7) passe par le premier trou (3) et est placée sur une surface externe de la section encerclant la taille (2),
l'autre partie d'extrémité de la bande de protection pour les douleurs lombaires (7) passe par le deuxième trou (4) et est placée sur une surface externe de la section encerclant la taille (2), et
la partie d'extrémité de la bande de protection pour les douleurs lombaires (7) est fixée de manière détachable sur l'autre partie d'extrémité de la bande de protection pour les douleurs lombaires (7),
**caractérisé en ce que** :
la bande de protection pour les douleurs lombaires (7) n'est pas fixée directement sur le vêtement pour partie inférieure du corps.

2. Vêtement pour partie inférieure du corps selon la revendication 1, ayant un moyen pour fixer de manière détachable la partie de protection de taille (8) sur le côté interne arrière de la section encerclant la taille (2).

3. Vêtement pour partie inférieure du corps selon la revendication 2, dans lequel le moyen de fixation a un moyen pour ajuster une position de fixation de la partie de protection de taille (8).

4. Vêtement pour partie inférieure du corps selon l'une quelconque des revendications 1 à 3, ayant au moins une boucle de ceinture (6) sur la section encerclant la taille (2).

5. Vêtement pour partie inférieure du corps selon la revendication 4, dans lequel une largeur de la boucle de ceinture (6) dans une direction encerclant la taille est de 1 cm à 5 cm, et une largeur de la boucle de ceinture (6) dans une direction verticale par rapport à la direction d'encerclement de la taille est de 2,5 cm à 20,5 cm.

6. Vêtement pour partie inférieure du corps selon l'une quelconque des revendications 1 à 5, ayant un troisième trou (16) et un quatrième trou (17) sur la section encerclant la taille (2), dans lequel le troisième trou (16) et le quatrième trou (17) sont positionnés plus à proximité d'un côté arrière de la section encerclant la taille (2) que le premier trou (3) et le deuxième trou (4).

7. Vêtement pour partie inférieure du corps selon la revendication 6, dans lequel une première surface de la bande de protection pour les douleurs lombaires (7) se trouve face à la surface interne de la section encerclant la taille (2),
la bande de protection pour les douleurs lombaires a une première partie de ceinture (28) positionnée au niveau du côté gauche de la partie de protection de taille (8, 14) se trouvant face à la première surface de la bande de protection pour les douleurs lombaires (7), une seconde partie de ceinture (29) positionnée du côté droit de la partie de protection de taille (8, 14) se trouvant face à la première surface de la partie de protection pour les douleurs lombaires (7), et une première ceinture auxiliaire (22) et une seconde ceinture auxiliaire (23) sur la première surface de la bande de protection pour les douleurs lombaires (7),
la première partie de ceinture (28) a une cinquième partie de fixation (19),
la seconde partie de ceinture (29) a une sixième partie de fixation (20),
une première partie d'extrémité de la première ceinture auxiliaire (22) est fixée sur la partie de protection de taille (8, 14) et sa seconde partie d'extrémité passe par le troisième trou (16) et est placée sur la surface externe de la section encerclant la taille (2),
une première partie d'extrémité de la seconde ceinture auxiliaire (23) est fixée sur la partie de protection de taille (8, 14) et sa seconde partie d'extrémité passe par le quatrième trou (17) et est placée sur la surface externe de la section encerclant la taille (2),
la seconde partie d'extrémité de la première ceinture auxiliaire (22) a une septième partie de fixation (24) qui est fixée de manière détachable sur la cinquième partie de fixation (19) ou la sixième partie de fixation (20), et
la seconde partie d'extrémité de la seconde ceinture auxiliaire (23) a une huitième partie de fixation (25) qui est fixée de manière détachable sur la cinquième partie de fixation (19) ou la sixième partie de fixation (20).

8. Vêtement pour partie inférieure du corps selon l'une quelconque des revendications 1 à 7, ayant une partie froncée (27) sur la totalité ou une partie de la section encerclant la taille (2).

9. Vêtement pour partie inférieure du corps selon la revendication 8, dans lequel une largeur de la partie froncée (27) dans la direction encerclant la taille est de 2 cm ou plus.
